# EUROPEAN PATENT APPLICATION

(11) **EP 1 637 532 A1**
(43) Date of publication of application: **22.03.2006**
(21) Application number: 04729197.6
(22) Date of filing: 23.04.2004
(51) Int. Cl.: C07D 487/14, A61K 31/519, A61P 3/10, A61P 9/10, A61P 13/12, A61P 25/00, A61P 27/02

(54) **FUSED PYRIMIDINE DERIVATIVE**

(30) Priority: 25.04.2003 JP 2003121287
(71) Applicant: KYOWA HAKKO KOGYO CO., LTD., Chiyoda-ku, Tokyo 100-8185 (JP)
(72) Inventor: NAKAJIMA, Takao, SUNTO-GUN, SHIZUOKA 411-8731 (JP); UENO, Kimihisa, SUNTO-GUN, SHIZUOKA 411-8731 (JP); NOMOTO, Yuji, SUNTO-GUN, SHIZUOKA 411-8731 (JP); MATSUMOTO, Yuichi, SUNTO-GUN, SHIZUOKA 411-8731 (JP); YANO, Hiroshi, SUNTO-GUN, SHIZUOKA 411-8731 (JP); NAKANISHI, Satoshi, Machida-shi, TOKYO 194-8533 (JP); TAKASAKI, Kotaro, FUKUOKA-SHI, FUKUOKA 810-0034 (JP); KUSAKA, Hideaki, SUNTO-GUN, SHIZUOKA 411-8731 (JP)
(74) Representative: Casalonga, Axel
(86) International application number: PCT/JP2004/005890
(87) International publication number: WO 2004/096812

(57) **Abstract**

The present invention provides a fused pyrimidine derivative having an insulin secretion stimulating activity represented by Formula (I): 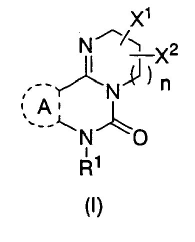 {wherein R¹ represents a hydrogen atom, lower alkyl, or the like; n represents an integer of 0 to 3; and X¹ and X² may be the same or different and each represents a hydrogen atom, lower alkyl, or the like; and formula (II): 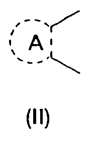 represents formula (III): 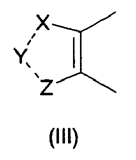 [wherein X--Y--Z represents R²C=CR³-NR⁴ (wherein R², R³ and R⁴ may be the same or different and each represents a hydrogen atom, lower alkyl, or the like)]}, or a pharmaceutically acceptable salt thereof.

## Description

### Technical Field

The present invention relates to a fused pyrimidine derivative having an insulin secretion stimulating activity or a pharmaceutically acceptable salt thereof.

### Background Art

Diabetes is caused by a metabolic disorder, mainly of glycometabolism, due to a deficiency of insulin secretion or a decrease of sensitivity of a target cell for insulin, and is characterized by causing hyperglycemia. Long-term hyperglycemia causes serious complications in various organs and nerves, i.e. retinopathy, nephropathy, neuropathy and the like, which are mainly caused by angiopathy. Therefore, it is extremely important in treatment of diabetes to control blood glucose levels within the normal revel, and the means of such controlling has been studied for a long time.

Among the types of diabetes, in the type which gradually develops and does not necessarily require insulin therapy as a life-sustaining treatment (non-insulin dependent diabetes mellitus (NIDDM)), blood glucose levels can be controlled by a combination of exercise therapy and drug therapy. As the drugs, insulin secretagogues, one of oral blood glucose-lowering agents, are widely used in clinicals. However, since every currently available insulin secretagogues promotes insulin secretion independently on blood glucose levels, they cause problems of severe hypoglycemia or insufficient control of blood glucose if doses are not appropriate, and are not fully satisfactory drugs. If blood glucose-lowering agents which is capable of enhancing insulin secretion dependently on blood glucose levels can be provided, the agents are expected to be extremely useful for controlling the blood glucose levels of diabetes patients because the risk of hypoglycemia due to overdose can be avoided.

On the other hand, with regard to fused pyrimidine derivatives, compounds represented by formula (A): 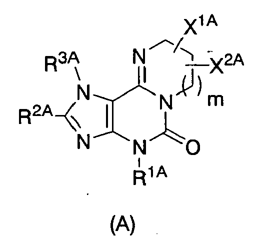 (wherein R^{1A} represents a hydrogen atom, lower alkyl, substituted or unsubstituted aralkyl, substituted or unsubstituted aryl, or substituted or unsubstituted aromatic heterocyclic group; R^{2A} represents a hydrogen atom, lower alkyl, substituted or unsubstituted aryl, or substituted or unsubstituted aromatic heterocyclic group; R^{3A} represents a hydrogen atom, lower alkyl, or substituted or unsubstituted aralkyl; X^{1A} and X^{2A} may be the same or different and each represents a hydrogen atom, lower alkyl, substituted or unsubstituted aralkyl, or substituted or unsubstituted aryl; and m represents an integer of 0 to 3), are known to have a diuretic effect, a mild anti-asthmatic effect, an anti-demential effect, a bronchodilatory effect, an anti-allergic effect or an anti-ulcer effect [See Japanese Published Unexamined Patent Application No. 204880/91; WO98/15555; J. Med. Chem., (1992) 35, p.3578; and J. Med. Chem., (1993) 36, p.2508], and are also known to have an insulin-secreting activity (see WO00/01388 and WO01/47931).

Further, the following Compound (B): 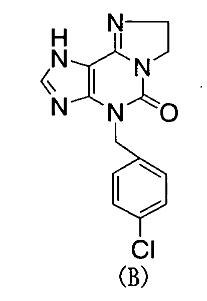 is known to have a mild bronchodilatory effect [see J. Med. Chem., (1980) 23, p.1188].

The following Compounds represented by formula (C): 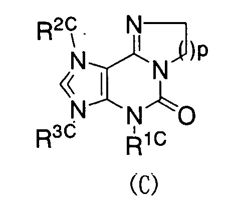 (wherein R^{1C}, R^{2C} and R^{3C} may be the same or different and each represents a hydrogen atom or C₁-C₆ alkyl optionally substituted with lower alkyloxy or acyl; and p represents an integer of 1 to 4), are known to show a type IV phosphodiesterase inhibitory effect (bronchodilatory effect) [see J. Med. Chem., (1997) 40, p.3248; and Japanese Published Unexamined Patent Application No. 158267/98].

The following Compounds represented by formula (D): 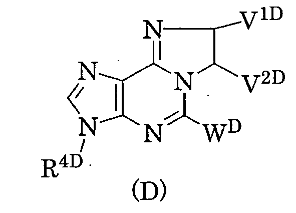 (wherein R^{4D} represents a hydrogen atom, phenyl or β-D-ribofuranosyl; W^{D} represents a hydrogen atom, C₁-C₄ alkyl or C₁-C₄ alkoxy; V^{1D} represents aralkyl; V^{2D} represents a hydrogen atom or phenyl; and when V^{2D} is phenyl V^{1D} may represent C₁-C₆ alkyl), are known to have an adenosine antagonistic effect (see European Patent Publication No. 390111).

### Disclosure of Invention

It is an object of the present invention to provide a fused pyrimidine derivative having an insulin secretion stimulating activity or a pharmaceutically acceptable salt thereof.

The present invention relates to the following aspects
(1) to (17):
   (1). A fused pyrimidine derivative or a pharmaceutically acceptable salt thereof represented by Formula (I): 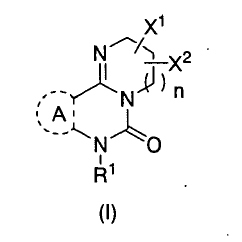 {wherein R¹ represents a hydrogen atom, substituted or unsubstituted lower alkyl, substituted or unsubstituted aralkyl, substituted or unsubstituted aryl, or substituted or unsubstituted aromatic heterocyclic group; n represents an integer of 0 to 3; X¹ and X² may be the same or different and each represents a hydrogen atom, substituted or unsubstituted lower alkyl, substituted or unsubstituted aralkyl, substituted or unsubstituted aryl, or substituted or unsubstituted aromatic heterocyclic group; and formula (II): 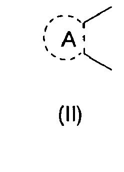 represents formula (III): 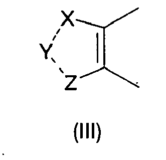 [wherein X--Y--Z represents R²C=CR³-NR⁴ (wherein R², R³ and R⁴ may be the same or different and each represents a hydrogen atom, substituted or unsubstituted lower alkyl, substituted or unsubstituted aralkyl, substituted or unsubstituted aryl, or substituted or unsubstituted aromatic heterocyclic group), R²C=N-NR⁴ (wherein R² and R⁴ have the same meanings as defined above, respectively), R⁴N-CR³=CR² (wherein R², R³ and R⁴ have the same meanings as defined above, respectively), or R⁴N-N=CR² (wherein R² and R⁴ have the same meanings as defined above, respectively)] or formula (IV): 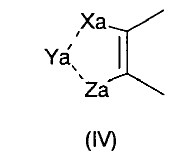 [wherein Xa--Ya--Za represents R²HC-NR³-CHR⁴ (wherein R², R³ and R⁴ have the same meanings as defined above, respectively), R²HC-NR³-NH (wherein R² and R³ have the same meanings as defined above, respectively), or NH-NR³-CHR⁴ (wherein R³ and R⁴ have the same meanings as defined above, respectively)]}.
   (2). The fused pyrimidine derivative or a pharmaceutically acceptable salt thereof according to the above (1), wherein R¹ is substituted or unsubstituted lower alkyl.
   (3). The fused pyrimidine derivative or a pharmaceutically acceptable salt thereof according to the above (1) or (2), wherein R³ is substituted or unsubstituted aryl, or substituted or unsubstituted lower alkyl.
   (4). The fused pyrimidine derivative or a pharmaceutically acceptable salt thereof according to any one of the above (1) to (3), wherein X¹ is substituted or unsubstituted aralkyl and X² is a hydrogen atom.
   (5). A pharmaceutical composition comprising the fused pyrimidine derivative or a pharmaceutically acceptable salt thereof according to any one of the above (1) to (4) as an active ingredient.
   (6). A therapeutic agent for diabetes comprising the fused pyrimidine derivative or a pharmaceutically acceptable salt thereof according to any one of the above (1) to (4) as an active ingredient.
   (7). A preventive and/or therapeutic agent for diabetic complications comprising the fused pyrimidine derivative or a pharmaceutically acceptable salt thereof according to any one of the above (1) to (4) as an active ingredient.
   (8). A blood glucose-lowering agent comprising the fused pyrimidine derivative or a pharmaceutically acceptable salt thereof according to any one of the above (1) to (4) as an active ingredient.
   (9). An insulin secretagogue comprising the fused pyrimidine derivative or a pharmaceutically acceptable salt thereof according to any one of the above (1) to (4) as an active ingredient.
   (10). Use of the fused pyrimidine derivative or a pharmaceutically acceptable salt thereof according to any one of the above (1) to (4) for a manufactur of a therapeutic agent for diabetes.
   (11). Use of the fused pyrimidine derivative or a pharmaceutically acceptable salt thereof according to any one of the above (1) to (4) for a manufactur of a preventive and/or therapeutic agent for diabetic complications.
   (12). Use of the fused pyrimidine derivative or a pharmaceutically acceptable salt thereof according to any one of the above (1) to (4) for a manufactur of a blood glucose-lowering agent.
   (13). Use of the fused pyrimidine derivative or a pharmaceutically acceptable salt thereof according to any one of the above (1) to (4) for a manufactur of an insulin secretagogue.
   (14). A method for treating diabetes, which comprises administering an effective amount of the fused pyrimidine derivative or a pharmaceutically acceptable salt thereof according to any one of the above (1) to (4).
   (15). A method for preventing and/or treating diabetic complications, which comprises administering an effective amount of the fused pyrimidine derivative or a pharmaceutically acceptable salt thereof according to any one of the above (1) to (4).
   (16). A method for lowering blood glucose levels, which comprises administering an effective amount of the fused pyrimidine derivative or a pharmaceutically acceptable salt thereof according to any one of the above (1) to (4).
   (17). A method for stimulating secretion of insulin which comprises administering an effective amount of the fused pyrimidine derivative or a pharmaceutically acceptable salt thereof according to any one of the above (1) to (4).

The compounds represented by Formula (I) are referred to as Compound (I). The compounds having the other formula numbers are referred to in the same manner.

In the definition of each group in Formula (I), examples of the lower alkyl include linear or branched alkyls having 1 to 10 carbon atoms, or cyclic alkyls having 3 to 12 carbon atoms, specifically, methyl, ethyl, n-propyl, isopropyl, n-butyl, isobutyl, sec-butyl, tert-butyl, n-pentyl, neopentyl, n-hexyl, n-heptyl, n-octyl, n-nonyl, n-decyl, cyclopropyl, cyclobutyl, cyclopentyl, cyclohexyl, cycloheptyl, cyclooctyl, noradamantyl, adamantly and the like. Examples of alkylene moiety of the aralkyl are the foregoing linear or branched alkyls having one hydrogen atom removed therefrom.

Examples of the aryl and the aryl moiety of the aralkyl include monocyclic aromatic ring groups or bicyclic- to penta-cyclic fused aromatic ring groups having 6 to 14 carbon atoms, and preferably monocyclic aromatic ring groups having 6 to 8 carbon atoms or 3 to 8-membered bicyclic- to penta-cyclic fused aromatic ring groups. The fused aromatic ring groups may contain saturated carbocyclic rings. Specific examples include phenyl, naphthyl, pentalenyl, indenyl, anthryl, phenanthryl, indanyl, indacenyl, 1,2,3,4-tetrahydronaphthyl, 6,7,8,9-tetrahydro-5H-benzocycloheptyl and the like.

Examples of the aromatic heterocyclic group include 5-membered or 6-membered monocyclic aromatic heterocycles having at least one heteroatom selected from a nitrogen atom, an oxygen atom and a sulfur atom; and 3 to 8-membered bicyclic- or tri-cyclic fused aromatic heterocyclic groups having at least one heteroatom selected from a nitrogen atom, an oxygen atom and a sulfur atom. Specific examples include furyl, thienyl, pyrrolyl, imidazolyl, pyrazolyl, triazolyl, tetrazolyl, oxazolyl, thiazolyl, pyridyl, pyrazinyl, pyrimidinyl, pyridazinyl, triazinyl, indolyl, indazolyl, benzimidazolyl, benzoxazolyl, benzothiazolyl, quinolyl, isoquinolyl, phthalazinyl, naphthylidinyl, quinoxalinyl, quinazolinyl, cinnolinyl, purinyl and the like.

Examples of the substituent of the substituted aryl, the substituted aromatic heterocyclic group and the substituted aralkyl, which may be the same or different and in number of 1 to 3, include substituted or unsubstituted lower alkyl, substituted or unsubstituted lower alkenyl, substituted or unsubstituted lower alkynyl, substituted or unsubstituted lower aralkyl, substituted or unsubstituted aryl, hydroxy, substituted or unsubstituted lower alkoxy, substituted or unsubstituted aralkyloxy, substituted or unsubstituted aryloxy, substituted or unsubstituted aroyl, substituted or unsubstituted lower alkoxycarbonyl, substituted or unsubstituted lower alkylthio, substituted or unsubstituted lower alkylsulfonyl, carboxy, mono- or di-lower alkyl substituted carbamoyl, substituted or unsubstituted lower alkanoyl, halogen, nitro, amino, mono-or di-lower alkyl substituted amino, cyano and the like. Herein, examples of the lower alkenyl include linear or branched alkenyls having 2 to 6 carbon atoms, specifically, vinyl, allyl, 1-propenyl, methacryl, butenyl, crotyl, pentenyl, hexenyl and the like. Examples of the lower alkynyl include linear or branched alkynyls having 2 to 6 carbon atoms, specifically, ethynyl, propynyl, butynyl, pentynyl, hexynyl and the like. The numbers of the unsaturated bond in the lower alkenyl and the lower alkynyl are preferably, but not limited to, one. The lower alkyl and the lower alkyl moieties of the lower alkoxy, the lower alkoxycarbonyl, the lower alkylthio, the lower alkylsulfonyl, the lower alkanoyl, the mono- or di-lower alkyl substituted carbamoyl, and the mono- or di-lower alkyl substituted amino have the same meanings as the lower alkyl defined above. The alkylene moieties of the aralkyl and the aralkyloxy have the same meanings as the alkylene defined above. The aryl moieties of the aralkyl, the aralkyloxy, the aryl, the aryloxy and the aroyl have the same meanings as the aryl defined above. Examples of the halogen include a fluorine atom, a chlorine atom, a bromine atom and an iodine atom. Examples of substituents of the substituted lower alkyl, the substituted lower alkenyl, the substituted lower alkynyl, the substituted aralkyl, the substituted aryl, the substituted lower alkoxy, the substituted aralkyloxy, the substituted aryloxy, the substituted aroyl, the substituted lower alkoxycarbonyl, the substituted lower alkylthio, the substituted lower alkylsulfonyl and the substituted lower alkanoyl, which may be the same or different and in number of 1 to 3, include hydroxy, halogen which has the same meaning as defined above, carboxy, sulfo, phospho, and esters derived from these acidic groups (e.g. lower alkylester, aralkylester, arylester and the like; and the lower alkyl moieties, the aralkyl moieties and the aryl moieties of these esters have the same meanings as the lower alkyl, the aralkyl and the aryl defined above, respectively). In the di-lower alkyl substituted carbamoyl and the di-lower alkyl substituted amino, two lower alkyls which bind to the carbamoyl or the amino, respectively may be the same or different.

Examples of the substituent of the the substituted lower alkyl, which may be the same or different and in number of 1 to 3, include lower alkoxy, halogen, cyano, hydroxy, trifluoromethanesulfonyloxy, toluenesulfonyloxy, lower alkoxycarbonyl, carboxy, lower alkylsulfonyloxy, substituted or unsubstituted heterocyclic group, -NR⁵R⁶ (wherein R⁵ and R⁶ may be the same or different and each represents a hydrogen atom, lower alkyl, aryl or aralkyl; or R⁵ and R⁶ form heterocyclic group together with the adjacent nitrogen atom), and the like. Examples of the heterocyclic group include aromatic heterocyclic group and alicyclic heterocyclic group. The aromatic heterocyclic group has the same meaning as defined above. Examples of the alicyclic heterocyclic group include 5-membered or 6-membered monocyclic alicyclic heterocyclic group containing at least one heteroatom selected from a nitrogen atom, an oxygen atom and a sulfur atom, and 3 to 8-membered bicyclic- or tri-cyclic fused alicyclic heterocyclic group containing at least one heteroatom selected from a nitrogen atom, an oxygen atom and a sulfur atom. Specific examples include pyrrolidinyl, 2,5-dioxopyrolydinyl, thiazolidinyl, oxazolidinyl, piperidinyl, piperazinyl, homopiperadinyl, morpholinyl, thiomorpholinyl, tetrahydropyranyl, tetrahydrothiopyranyl, tetrahydrofuryl, tetrahydroquinolyl, tetrahydroisoquinolyl, tetrahydroquinoxalinyl, octahydroquinolyl, dihydroindolyl, 1,3-dioxoisoindolinyl and the like. Examples of the heterocyclic group formed with the adjacent nitrogen atom include pyrrolidinyl, thiazolidinyl, oxazolidinyl, piperidino, homopiperidino, piperazinyl, homopiperazinyl, morpholino, thiomorpholino, tetrahydroquinolyl, tetrahydroisoquinolyl, octahydroquinolyl, benzimidazolyl, indazolyl, indolyl, isoindolyl, purinyl, dihydroindolyl, pyrrolyl, pyrazolyl, triazolyl, tetrazolyl, imidazolyl and the like. The lower alkyl moieties of the lower alkoxy, the lower alkoxycarbonyl, the lower alkylsulfonyloxy and the lower alkyl have the same meanings as the lower alkyl defined above. The aryl and the aryl moieties of the aralkyl have the same meanings as the aryl defined above. The alkylene moiety of the aralkyl has the same meaning as the alkylene defined above. The halogen has the same meaning as defined above. The substituents in the substituted heterocyclic group has the same meanings as the substituents in the substituted aromatic heterocyclic grpup defined above.

In Formula (I), X¹ and X² can be each substituted at any positions on the ring without any limitations. When X¹ or X² is the substituent other than a hydrogen atom, the carbon atom to which the substituent binds may have either an S-configuration or an R-configuration. Preferably, n is 0 to 1, more preferably, n is 0.

The pharmaceutically acceptable salts of Compound (I) include acid addition salts, metal salts, ammonium salts, organic amine addition salts, amino acid addition salts and the like. Examples of the pharmaceutically acceptable acid addition salts include inorganic acid salts such as a hydrochloride, a sulfate, and a phosphate; and organic acid salts such as an acetate, a maleate, a fumarate, a tartrate, and a citrate. Examples of the pharmaceutically acceptable metal salts include alkali metal salts such as a sodium salt and a potassium salt, alkaline earth metal salts such as a magnesium salt and a calcium salt, aluminium salts, zinc salts and the like. Examples of the pharmaceutically acceptable organic amine addition salts include addition salts of morpholine, piperidine or the like. Examples of the pharmaceutically acceptable amino acid addition salts include addition salts of lysine, glycine, phenylalanine or the like.

Compound (I) or a pharmaceutically acceptable salt thereof may exist as a form of hydrates or solvates, and these adducts are encompassed within the present invention. The solvents used to form the solvates are not paticulary limited, so long as they are pharmaceutically acceptable. Example of such solvents are ethanol, acetone and the like. Compound (I) may have one or more asymmetric carbons; and any of opyical isomers and diastereomers in a pure form, any mixtures of these isomers in any ratio, racemates and the like are also encompassed within the present invention. When Compound (I) contains a double bond, the configuration may be either a Z-configuration or an E-configuration. When a tautomer can exist in Compound (I), any tautomer is included. Thus, all possible isomers and mixtures thereof in any ratio are encompassed within the present invention.

Methods for preparing Compound (I) will now be described.

The preparation of Compound (I) can be performed according to known methods [e.g. Japanese Unexamined Patent Application Publication No. 204880/91; WO98/15555; J. Med. Chem., (1992) 35, p.3578; J. Med. Chem., (1993) 36, p.2508; and J. Heterocyclic Chem., (1993) 30, p.241].

Compound (I) can be prepared by persons skilled in the art according to the methods discribed in the above-mentioned references or preparing methods specifically disclosed in the specification, or by modifying reagents and reaction materials used in the methods and appropriately modifying or altering the methods if necessary.

In preparing methods described below, when a defined group changes under reaction conditions or is unsuitable for the method, the method can be readily performed by utilizing a method which is generally used in organic synthetic chemistry, for example, protection and deprotection of the functional group (see, for example, T. W. Greene, Protective Groups in Organic Synthesis, John Wiley & Sons, Inc., 1981).

### Preparing method 1

Compound (I) can be prepared according to the following reaction steps: 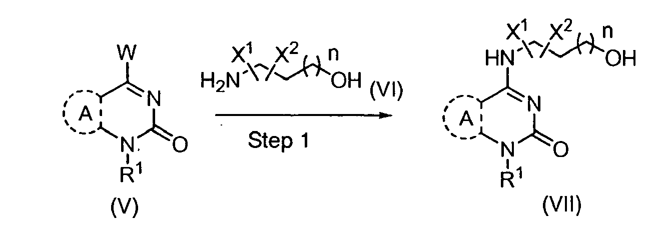 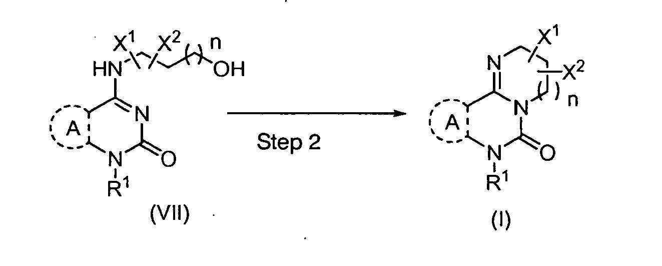 (wherein R¹, 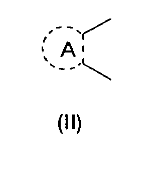 X¹, X² and n have the same meanings as defined above, and W represents a leaving group).

Examples of the leaving group include halogen, methylthio, methanesulfonyloxy, toluenesulfonyloxy, trifluoromethanesulfonyloxy and the like. The halogen has the same meaning as defined above.

### Step 1

Compound (VII) can be prepared by reacting Compound (V) with 1 to 10 equivalents of, preferably, 2 to 5 equivalents of Compound (VI) without solvent or in a suitable solvent, in the presence of 1 to 10 equivalents of, preferably, 1 to 3 equivalents of base if necessary. Examples of the solvent include alcohols such as methanol and ethanol; ketones such as acetone and methyl ethyl ketone; aromatic hydrocarbons such as toluene and xylene; halogenated hydrocarbons such as dichloroethane, 1,1,2,2-tetrachloroethane and dichlorobenzene; pyridine; N,N-dimethylformamide; N,N-dimethylacetamide; N-methylpyrrolidinone; N,N'-dimethylimidazolidine-2-one; dimethylsulfoxide and the like. These solvents are used alone or in combination. Examples of the base include triethylamine, N,N-diisopropylethylamine, 4-dimethylaminopyridine and the like. The reaction is usually performed at a temperature between 50°C and 180°C for 5 minutes to 24 hours.

Starting material, Compound (V), can be prepared by known methods or modified methods thereof [e.g. European Patent Publication No. 736569; Chem. Pharm. Bull., (1980), 28, p.1636; Chem. Pharm. Bull., (1972) 20, p.399; J. Chem. Soc. Parkin I, (1982) p.277].

Starting material, Compound (VI), can be prepared by known methods or modified methods thereof (e.g. WO00/01388, WO01/47931

### Step 2

Compound (I) can be prepared by treatment of Compound (VII) with one equivalent to large excess amount of, preferably, large excess amount of a halogenating agent such as thionyl chloride, phosphorus oxychloride or the like, without solvent or in a suitable solvent; by treatment of Compound (VII) with an inorganic acid such as a hydrochloric acid, a hydrobromic acid, a hydroiodic acid or a phosphoric acid; or by treatment of Compound (VII) with 1 to 5 equivalents of, preferably, 1 to 2 equivalents of a sulfonylating agent such as benzenesulfonyl chloride, p-toluenesulfonyl chloride, methanesulfonyl chloride, trifluoromethanesulfonyl chloride or the like, in the presence of 1 to 10 equivalents of, preferably, 1 to 5 equivalents of an organic base (e.g. triethylamine, diisopropylethylamine, pyridine and the like) or an inorganic base (e.g. potassium carbonate, sodium carbonate, potassium hydrogen carbonate, sodium hydrogen carbonate, potassium hydroxide, sodium hydroxide and the like). Examples of the solvent include halogenated hydrocarbons such as methylene chloride, chloroform and dichloroethane; tetrahydrofurane; N,N-dimethylformamide; dimethylsulfoxide and the like. These solvents are used alone or in combination. The reaction is usually performed at a temperature between -10°C and 150°C, preferably, at a temperature between 50°C and 70°C for 5 minutes to 24 hours.

Intermediate compounds and desired compounds prepared by these preparing methods can be isolated and purified by purification procedures generally used in organic synthetic chemistry, for example, neutralization, filtration, extraction, washing, drying, concentration, recrystallization, various types of chromatography and the like. The intermediate compounds may also be used in a subsequent reaction without purification. The salts of Compound (I) can be prepared by dissolving or suspending Compound (I) in a free form in a proper solvent, and then adding an acid or a base suitable for forming the salts. Then, the salts are isolated or purified if necessary. A desired salt can be prepared by converting a desired product obtained in a salt form into a free form, and then converting the free form into the desired salt.

Table 1 shows examples of Compound (I) prepared by the above preparing method.

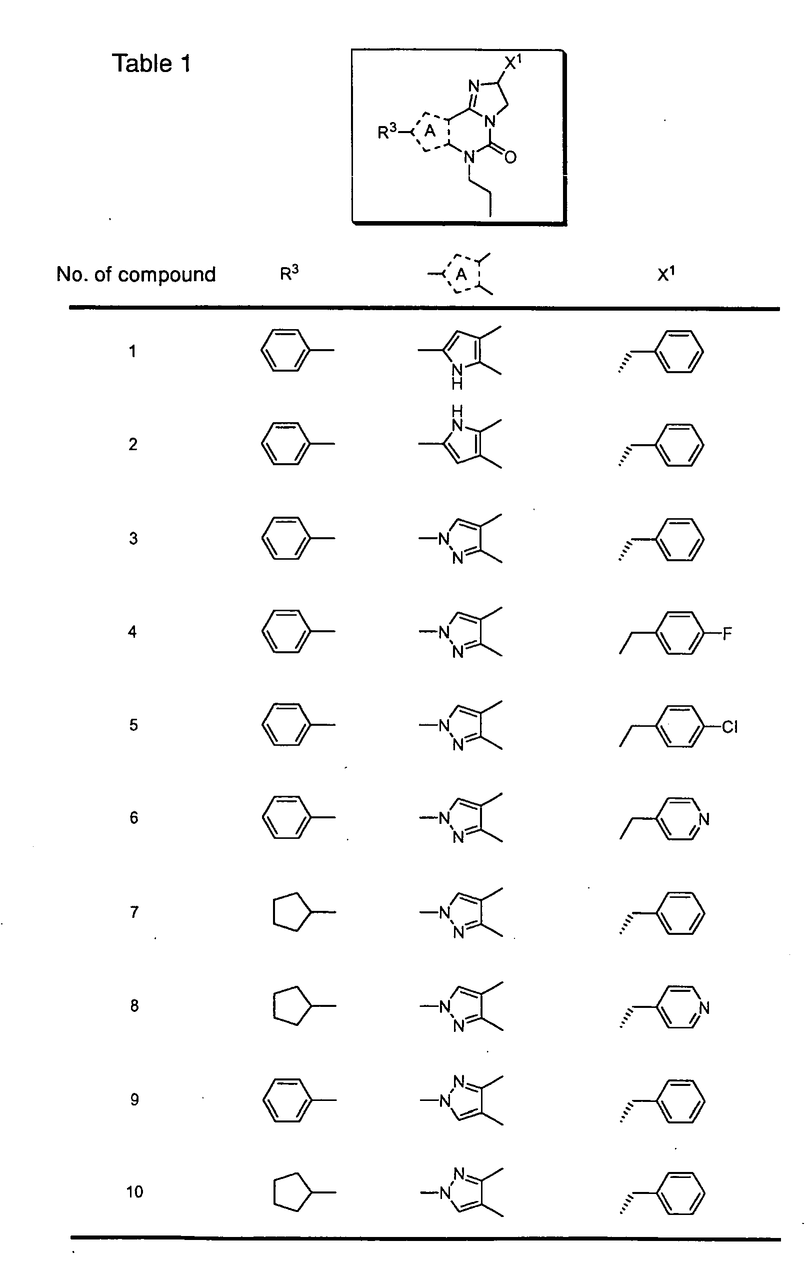

Since Compound (I) or a pharmaceutically acceptable salt thereof exhibits an insulin secretion stimulating effect in cultured β cells, it is useful as active ingredients of medicament for treating diabetes, and is also useful as active ingredients of medicament for preventing and/or treating diabetic complications, e.g. retinopathy, nephropathy, neuropathy and the like. With regard to the active ingredient of these medicaments, one or more substance(s) selected from the group consisting of Compound (I) and a pharmaceutically acceptable salt thereof, hydrates thereof, and solvates thereof can be used. The substance(s) can be administered alone, but, generally, the substance(s) are preferably provided as various pharmaceutical preparations. The pharmaceutical preparations are administered to humans and animals.

The pharmaceutical preparations according to the present invention can contain Compound (I) or a pharmaceutically acceptable salt thereof as an active ingredient alone or in combination with any active ingredient for other treatments. These pharmaceutical preparations can be prepared by mixing the active ingredient with one or more pharmacologically acceptable additives according to any method that is widely known in the technical field of pharmaceutical preparations.

The administration route which is the most effective for treating is preferably used, specifically, an oral route and a parenteral route, e.g. an intravenous route, are included.

Examples of administration form include tablets, powder, granules, syrup, injections and the like.

An administration form suitable for the oral route, for example, tablets, can be prepared by using an excipient such as lactose, a disintegrator such as cornstarch, a lubricant such as magnesium stearate, a binder such as hydroxypropyl cellulose, and the like.

An administration form suitable for the parenteral route, for example, injections, can be prepared by using a salt solution, a glucose solution, or a mixture of a salt solution and a glucose solution.

Dose and frequency of administration of Compound (I) or a pharmaceutically acceptable salt thereof depend on the administration form, age and weight of the patient, and property or severity of a symptom to be treated. Generally, in the oral administration, 0.01 mg to 1 g, preferably, 0.05 to 50 mg is administered to an adult once or several times a day. In the parenteral administration such as intravenous administration, 0.001 to 100 mg, preferably, 0.01 to 10 mg is administered to an adult once or several times a day. However, these doses and frequencies of administration vary by the various conditions described above.

### Best Mode for Carrying Out the Invention

The present invention will now be specifically described with reference to EXAMPLES, but the present invention is not limited to the following EXAMPLES.

### EXAMPLE 1: (R)-2-Benzyl-2,3-dihydro-8-phenyl-6-propyl-7H-imidazo[1,2-c]pyrrolo[3,2-e]pyrimidin-5(6H)-one (Compound 1)

Compound A (1.00 g, 3.34 mmol) prepared in Reference Example 1 and (R)-phenylalaninol (1.01 g, 6.69 mmol) were suspended in chloroform (1 mL), and the mixture was stirred at 90°C for 15 minutes and then 150°C for 3 hours. The reaction mixture was air-cooled to room temperature and purified by silica gel column chromatography (chloroform/methanol = 50/1 to 25/2) to give (R)-4-(1-hyroxy-3-phenylpropane-2-amino)-6-phenyl-1-propyl-7H-pyrrolo[2,3-d]pyrimidin-2(1H)-one (Compound 1a: 1.10 g, 82%).

Compound 1a (1.10 g, 2.74 mmol) was dissolved in thionyl chloride (10 mL), and the mixture was stirred at 60°C for 1 hour. The solvent was evaporated from the reaction mixture under reduced pressure, and saturated aqueous sodium hydrogen carbonate (50 mL) was added to the residue, then the mixture was extracted with chloroform (50 mL). The organic layer was washed with saturated brine (50 mL) and dried over anhydrous magnesium sulfate. The organic layer was concentrated and purified by silica gel column chromatography(chloroform to chloroform/methanol = 50/1) to give (R)-2-benzyl-9-chloro-2,3-dihydro-8-phenyl-6-propyl-7H-imidazo[1,2-c]pyrrolo[3,2-e]pyrimidin-5(6H)-one (Compound 1b: 360 mg, 32%).

Compound 1b (350 mg, 0.840 mmol) was dissolved in ethanol (200 mL), and to the mixture was added 1.0 g of 10% palladium-carbon (containing 50% water). The mixture was stirred under a hydrogen atmosphere at room temperature overnight. The reaction mixture was filtered through Celite. The filtrate was concentrated and purified by silica gel column chromatography (chloroform/methanol = 50/3) to give the title compound (27.0 mg, 8%).
¹H-NMR(270 MHz, DMSO-d₆)δ 7.77 (2H, d, J = 7.3 Hz), 7.47 (2H, dd, J = 7.9, 7.3 Hz), 7.38-7.22 (6H, m), 6.91 (1H, s), 4.74 (1H, m), 4.21 (1H, dd, J = 11.0, 10.2 Hz), 4.11 (2H, t, J = 7.4 Hz), 3.93 (1H, dd, J = 11.0, 6.3 Hz), 3.05 (2H, d, J = 6.3 Hz), 1.74-1.50 (2H, m), 0.92 (3H, t, J = 7.4 Hz).
FABMS m/z: 385 (M + H)⁺.

### EXAMPLE 2: (R)-8-Benzyl-7,8-dihydro-2-phenyl-4-propyl-1H-imidazo[1,2-c]pyrrolo[2,3-e]pyrimidin-5(4H)-one (Compound 2)

Compound D (1.11 g, 3.71 mmol) prepared in Reference Example 2 and (R)-phenylalaninol (1.21 g, 8.00 mmol) were dissolved in a mixed solvent of chloroform (1 mL) and methanol (1 mL), and the mixture was stirred at 90°C for 10 minutes then 150°C for 1.5 hours. The reaction mixture was air-cooled to room temperature and purified by silica gel column chromatography (chloroform/methanol = 20/1 to 25/2) to give (R)-4-(1-hydroxy-3-phenylpropane-2-amino)-6-phenyl-1-propyl-5H-pyrrolo[3,2-d]pyrimidin-2(1H)-one (Compound 2a: 647 mg, 43%).

Compound 2a (510 mg, 1.27 mmol) was dissolved in chloroform (10 mL) and to the mixture were added pyridine (0.246 mL, 3.00 mmol) and methanesulfonyl chloride (0.234 mL, 3.00 mmol), then the mixture was stirred at room temperature for 4 hours. The reaction mixture was purified by silica gel column chromatography (chloroform to chloroform/methanol = 50/1) to give the title compound (270 mg, 55%).
¹H-NMR(270 MHz, DMSO-d₆)δ 7.89 (2H, d, J = 7.9 Hz), 7.57-7.43 (3H, m), 7.40-7.23 (5H, m), 6.95 (1H, s), 4.71 (1H, m), 4.11 (1H, dd, J = 10.6, 10.6 Hz), 3.84 (2H, t, J'= 6.3 Hz), 3.87-3.70 (1H, m), 3.11-2.95 (2H, m), 1.73-1.62 (2H, m), 0.91 (3H, t, J = 7.3 Hz).
FABMS m/z: 385 (M + H)⁺.

### EXAMPLE 3: (R)-2-Benzyl-2,3-dihydro-8-phenyl-6-propyl-8H-imidazo[1,2-c]pyrazolo[4,3-e]pyrimidin-5(6H)-one (Compound 3)

Compound H (500 mg, 1.73 mmol) prepared in Reference Example 3 was dissolved in 2-propanol (20 mL), and to the solution was added (R)-phenylalaninol (1.51 g, 10.0 mmol), then the mixture was heated under reflux overnight. The solvent was evaporated from the reaction mixture under reduced pressure, and the residue was purified by silica gel column chromatography (chloroform/methanol = 50/1 to 25/1) to give (R)-4-(1-hydroxy-3-phenylpropane-2-amino)-2-phenyl-7-propyl-2H-pyrazolo[3,4-d]pyrimidin-6(7H)-one (Compound 3a: 665 mg, 95%).

Compound 3a (660 mg, 1.63 mmol) was dissolved in thionylchloride (20 mL), and the mixture was stirred at 60°C for 1 hour. The solvent was evaporated from the reaction mixture under reduced pressure. To the residue was added saturated aqueous sodium hydrogen carbonate (50 mL), and the mixture was extaracted with chloroform (100 mL x 2). An organic layer was washed with saturated brine (100 mL) and dried over anhydrous magnesium sulfate. The organic layer was concentrated and purified by silica gel column chromatography (chloroform to chloroform/methanol = 100/1) to give the title compound (224 mg, 36%).
¹H-NMR(270 MHz, CDCl₃)δ 8.35 (1H, s), 7.68 (2H, d, J = 8.2 Hz), 7.51-7.45 (2H, m), 7.37-7.20 (6H, m), 4.58 (1H, m), 3.97 (2H, t, J = 7.4 Hz), 3.89 (1H, dd, J = 11.0, 9.9 Hz), 3.68 (1H, dd, J = 11.0., 7.3 Hz), 3.23 (1H, dd, J = 13.7, 5.3 Hz), 2.77 (1H, dd, J = 13.7, 8.9 Hz), 1.85-1.77 (2H, m), 0.99 (3H, t, J = 7.4 Hz).
EIMS m/z: 383 (M)⁺.

### EXAMPLE 4: 2-(4-Fluorobenzyl)-2,3-dihydro-8-phenyl-6-propyl-8H-imidazo[1,2-c]pyrazolo[4,3-e]pyrimidin-5(6H)-one (Compound 4)

Compound L (150 mg, 0.500 mmol) prepared in Reference Example 4 and (4-fluorophenyl)alaninol (169 mg, 1.00 mmol) were dissolved in chloroform (2 mL), and the mixture was stirred at 90°C for 10 minutes then at 150°C for 1.5 hours. The reaction mixture was air-cooled to room temperature and purified by silica gel column chromatography (chloroform/methanol = 100/1 to 20/1) to give 4-[1-hydroxy-3-(4-fluorophenyl)propane-2-amino]-2-phenyl-7-propyl-2H-pyrazolo[3,4-d]pyrimidin-6(7H)-one (Compound 4a: 163 mg, 77%).

Compound 4a (163 mg, 0.387 mmol) was dissolved in thionylchloride (5 mL), and the mixture was heated under reflux for 30 minutes. The solvent was evaporated from the reaction mixture under reduced pressure. To the residue were added saturated aqueous sodium hydrogen carbonate (30 mL) and ethyl acetate (5 mL). After being stirred at room temperature for 1 hour, the mixture was extracted with chloroform (30 mL). The organic layer was washed with saturated brine (10 mL) and dried over anhydrous magnesium sulfate. The organic layer was concentrated and purified by silica gel column chromatography (chloroform/methanol = 100/1 to 50/1) to give the title compound (20.0 mg, 19%).
¹H-NMR(270 MHz, CDCl₃)δ 8.33 (1H, s), 7.67 (2H, d, J = 8.4 Hz), 7.49 (2H, dd, J = 8.4, 7.4 Hz), 7.37 (1H, t, J = 7.4 Hz), 7.23 (2H, dd, J = 8.6, 5.1 Hz), 6.99 (2H, dd, J = 8.6, 8.6 Hz), 4.55 (1H, s), 4.00-3.87 (3H, m), 3.68 (1H, dd, J = 10.8, 7.0 Hz), 3.13 (1H, dd, J = 13.8, 5.7 Hz), 2.78 (1H, dd, J = 13.8, 8.1 Hz), 1.85-1.77 (2H, m), 0.99 (3H, t, J = 7.4 Hz).
EIMS m/z: 402 (M)⁺.

### EXAMPLE 5: 2-(4-Chlorobenzyl)-2,3-dihydro-8-phenyl-6-propyl-8H-imidazo[1,2-c]pyrazolo[4,3-e]pyrimidin-5(6H)-one (Compound 5)

The title compound (97.0 mg, 46%) was obtained by a method similar to that of EXAMPLE 4 by using Compound L (150 mg, 0.500 mmol) prepared in Reference Example 4 and (4-chlorophenyl)alaninol (150 mg, 0.500 mmol).
¹H-NMR(270 MHz, CDCl₃)δ 8.33 (1H, s), 7.67 (2H, d, J = 7.8 Hz), 7.48 (2H, dd, J = 7.8, 7.6 Hz), 7.34 (1H, t, J = 7.6 Hz), 7.27 (2H, d, J = 8.8 Hz), 7.20 (2H, d, J = 8.8 Hz), 4.54 (1H, m), 4.00-3.88 (3H, m), 3.64 (1H, dd, J = 13.5, 5.9 Hz), 3.12 (1H, dd, J = 13.5, 5.9 Hz), 2.79 (1H, dd, J = 13.5, 8.1 Hz), 1.85-1.77 (2H, m), 0.99 (3H, t, J = 7.4 Hz).
EIMS m/z: 419 (³⁷ClM)⁺, 417 (³⁵ClM)⁺.

### EXAMPLE 6: 2,3-Dihydro-8-phenyl-6-propyl-2-(4-picolyl)-8H-imidazo[1,2-c]pyrazolo[4,3-e]pyrimidin-5(6H)-one (Compound 6)

The title compound (174 mg, 46%) was obtained by a method similar to that of EXAMPLE 4 by using Compound L (300 mg, 1.00 mmol) prepared in Reference Example 4 and 2-amino-3-(4-pyridyl)-1-propanol (182 mg, 1.20 mmol) prepared by the method discribed in WO01/47931.
¹H-NMR(270 MHz, CDCl₃)δ 8.54 (2H, d, J = 6.1 Hz), 8.33 (1H, s), 7.68 (2H, d, J = 8.2 Hz), 7.49 (2H, dd, J = 8.2, 7.4 Hz), 7.35 (1H, t, J = 7.4 Hz), 7.22 (2H, d, J = 6.1 Hz), 4.60 (1H, m), 4.02-3.94 (3H, m), 3.64 (1H, dd, J = 11.2, 7.4 Hz), 3.11 (1H, dd, J = 13.6, 5.9 Hz), 2.86 (1H, dd, J = 13.6, 7.6 Hz), 1.86-1.77 (2H, m), 0.99 (3H, t, J = 7.4 Hz).

### EXAMPLE 7: (R)-2-Benzyl-8-cyclopentyl-2,3-dihydro-6-propyl-8H-imidazo[1,2-c]pyrazolo[4,3-e]pyrimidin-5(6H)-one hydrochloride (Compound 7)

The title compound in a free form (Compound 7a: 320 mg, 91%) was prepared by a method similar to that of EXAMPLE 4 by using Compound M (292 mg, 1.00 mmol) prepared in Reference Example 5 and (R)-phenylalaninol (227 mg, 1.50 mmol).

Compound 7a (320 mg, 0.850 mmol) was dissolved in ethyl acetate (4 mL), and to the mixture was added a 4 mol/L hydrogen chloride/ethyl acetate solution (2 mL), then the mixture was stirred at room temperature for 30 minutes. The solvent was evaporated from the reaction mixture under reduced pressure to give the title compound (288 mg, 82%).
¹H-NMR(270 MHz, DMSO-d₆)δ 8.61 (1H, s), 7.40-7.20 (5H, m), 4.95 (1H, m), 4.78 (1H, m), 4.20 (1H, dd, J = 11.1, 10.5 Hz), 3.95-3.75 (3H, m), 3.35 (2H, d, J = 6.8 Hz), 2.20-2.05 (2H, m), 1.95-1.75 (4H, m), 1.75-1.55 (4H, m), 0.86 (3H, t, J = 7.4 Hz).
ESIMS m/z: 378 (M + H)⁺.

### EXAMPLE 8: (R)-8-Cyclopentyl-2,3-dihydro-6-propyl-2-(4-picolyl)-8H-imidazo[1,2-c]pyrazolo[4,3-e]pyrimidin-5(6H)-one (Compound 8)

The title compound (148 mg, 40%) was obtained by a method similar to that of EXAMPLE 4 by using Compound M (292 mg, 1.00 mmol) prepared in Reference Example 5 and (R)-2-amino-3-(4-pyridyl)-1-propanol (182 mg, 1.20 mmol) prepared by the method discribed in WO01/47931.
¹H-NMR(270 MHz, CDCl₃)δ 8.52 (2H, d, J = 5.4 Hz), 7.82 (1H, s), 7.20 (2H, d, J = 5.4 Hz), 4.64-4.48 (2H, m), 3.96-3.85 (3H, m), 3.59 (1H, dd, J = 10.8, 7.0 Hz), 3.08 (1H, dd, J = 13.5, 5.7 Hz), 2.86 (1H, dd, J = 13.5, 7.6 Hz), 2.25-2.05 (2H, m), 2.05-1.90 (2H, m), 1.90-1.75 (2H, m), 1.75-1.60 (4H, m), 0.95 (3H, t, J = 7.4 Hz).
ESIMS m/z: 379 (M + H)⁺.

### EXAMPLE 9: (R)-8-Benzyl-7,8-dihydro-2-phenyl-4-propyl-2H-imidazo[1,2-c]pyrazolo[3,4-e]pyrimidin-5(4H)-one (compound 9)

The title compound (27.0 mg, 24%) was obtained by a method similar to that of EXAMPLE 4 by using Compound T (90.0 mg, 0.300 mmol) prepared in Reference Example 6 and (R)-phenylalaninol (227 mg, 1.50 mmol).
¹H-NMR(270 MHz, CDCl₃)δ 7.80 (2H, d, J = 8.2 Hz), 7.63 (1H, s), 7.48 (2H, dd, J = 8.2, 7.3 Hz), 7.36 (1H, t, J. = 7.3 Hz), 7.31-7.18 (5H, m), 4.67 (1H, m), 3.90 (1H, dd, J = 11.0, 10.2 Hz), 3.76 (2H, t, J = 7.6 Hz), 3.71 (1H, dd, J = 11.0, 7.6 Hz), 3.36 (1H, dd, J = 13.9, 5.0 Hz), 2.79 (1H, dd, J = 13.9, 9.2 Hz), 1.79-1.68 (2H, m), 0.99 (3H, t, J = 7.4 Hz).
FABMS m/z: 386 (M + H)⁺.

### EXAMPLE 10: (R)-8-Benzyl-2-cyclopentyl-7,8-dihydro-4-propyl-2H-imidazo[1,2-c]pyrazolo[3,4-e]pyrimidin-5(4H)-one hydrochloride (Compound 10)

The title compound in a free form (Compound 10a: 113 mg, 52%) was prepared by a method similar to that of EXAMPLE 4 by using Compound X (216 mg, 0.740 mmol) prepared in Reference Example 7 and (R)-phenylalaninol (227 mg, 1.50 mmol).

Compound 10a (113 mg, 0.299 mmol) was dissolved in ethyl acetate (10 mL), and to the mixture was added a 4 mol/L hydrogen chloride/ethyl acetate solution (3 mL). The mixture was stirred at room temperature for 30 minutes. The solvent was evaporated from the reaction mixture under reduced pressure to give the title compound (112 mg, 91%).
¹H-NMR(270 MHz, DM SO-d₆)δ 8.44 (1H, s), 7.36 (5H, m), 4.95 (1H, m), 4.82 (1H, m), 4.18 (1H, dd, J = 10.8, 10.8 Hz), 3.93 (1H, dd, J = 10.8, 6.8 Hz), 3.78 (2H, t, J = 6.8 Hz), 3.20-3.00 (2H, m), 2.25-2.15 (2H, m), 2.10-1.90 (2H, m), 1.90-1.50 (6H, m), 0.88 (3H, t, J = 7.3 Hz).
EIMS m/z: 378 (M + H)⁺.

### Reference Example 1: 4-Methylthio-6-phenyl-1-propyl-7H-pyrrolo[2,3-d]pyrimidin-2(1H)-one (Compound A)

Step 1: A mixture of phenacyl bromide (9.50 g, 50.0 mmol) and 6-amino-1-propyl-2,4(1H,3H)-pyrimidindione (8.45 g, 50.0 mmol) in acetic acid (50 mL) was stirred at 95°C for 5 hours. The reaction mixture was air-cooled and the solvent was evaporated under reduced pressure, then the residue was poured into water (200 mL). The mixture was filtrated to separate the filtrate and the precipitate, and the precipitate was washed with ethanol. The filtrate and the washings were combined and concentrated, then purified by silica gel column chromatography (chloroform/methanol = 100/1 to 20/1) to give 6-phenyl-1-propyl-7H-pyrrolo[2,3-d]pyrimidin-2,4(1H,3H)-dione (Compound B: 1.80 g, 13%).
¹H-NMR(270 MHz, DMSO-d₆)δ 11.50 (1H, br s), 10.84 (1H, br s), 7.71 (2H, d, J = 8.3 Hz), 7.44-7.38 (2H, m), 7.25 (1H, t, J = 6.9 Hz), 6.75 (1H, s), 3.96 (2H, t, J = 7.4 Hz), 1.73-1.59 (2H, m), 0.92 (3H, t, J = 7.3 Hz).

Step 2: Compound B (1.50 g, 5.58 mmol) prepared in Step 1 was suspended in pyridine (15 mL), and to the mixture was added phosphorus pentasulfide (2.48 g, 11.2 mmol), then the mixture was heated under reflux for 2 hours. The reaction mixture was air-cooled and poured into iced water. The mixture was filtrated to separate the filtrate and the precipitate, and the precipitate was washed with water and with 2 mol/L aqueous sodium hydroxide. The washings was adjusted to pH 3 by adding 4 mol/L hydrochloric acid. The resulting precipitate in the washings was collected by filtration and dried to give 3,4-dihydro-6-phenyl-1-propyl-4-thioxo-7H-pyrrolo[2,3-d]pyrimidin-2(1H)-one (Compound C: 1.32 g, 83%).

Compound C (1.28 g, 4.50 mmol) was dissolved in a mixed solvent of 0.5 mol/L aqueous sodium hydroxide (15 mL) and ethanol (7 mL). To the mixture was added iodomethane (0.311 mL, 5.00 mmol), and the mixture was stirred at room temperature for 1 hour. Ethanol was evaporated from the reaction mixture under reduced pressure, and the residue was adjusted to pH 3 by adding 4 mol/L hydrochloric acid. Then, the resulting precipitate was collected by filtration and dried to give the title compound (1.20 g, 89%) as a yellow solid.
¹H-NMR(270 MHz, DMSO-d₆)δ 11.68 (1H, br s), 7.75 (2H,-d, J = 7.3 Hz), 7.46-7.36 (2H, m), 7.26 (1H, t, J = 7.3 Hz), 6.72 (1H, s), 4.01 (2H, t, J = 7.4 Hz), 2.45 (3H, s), 1.69-1.60 (2H, m), 0.89 (3H, t, J = 7.3 Hz).

### Reference Example 2: 4-Methylthio-6-phenyl-1-propyl-5H-pyrrolo[3,2-d]pyrimidin-2(1H)-one (Compound D)

A mixed solvent of concentrated nitric acid (75 mL) and concentrated sulfuric acid (75 mL) was cooled in ice-brine bath to maintain the internal temperature at 5°C or less, and 6-methyl-1-propyl-2,4(1H,3H)-pyrimidindione (14.9 g, 89.0 mmol) prepared by the method discribed in J. Chem. Soc., (1959) p.1169 was added to the solvent, then the mixture was stirred for 1 hour at the same temperature. The reaction mixture was poured into iced water (375 mL), and the resalting precipitate was isolated by filtration. The precipitate was washed with water and dried to give 5-nitro-6-methyl-1-propyl-2,4(1H,3H)-pyrimidindione (Compound E: 14.3 g, 75%).

A mixture of Compound E (12.8 g, 60.0 mmol) and benzaldehyde (6.37 g, 60.0 mmol) was suspended in ethanol (300 mL), and to the mixture was added piperidine (6.00 mL, 60.0 mmol), then the mixture was heated under reflux for 5 hours. The reaction mixture was air-cooled, and the solvent was evaporated under reduced pressure. The residue was crystallized from ethanol to give 5-nitro-1-propyl-6-styryl-2,4(1H,3H)-pyrimidindione (Compound F: 13.9 g, 77%).

Compound F (13.5 g, 45.0 mmol) was suspended in formic acid (450 mL), and to the mixture was assed sodium dithionite (39.2 g, 225 mmol), then the mixture was heated under reflux overnight. The reaction mixture was air-cooled, and the solvent was evaporated under reduced pressure. Then to the residue was added hot water, and the resulting precipitate was isolated by filtration. The precipitate was dried and purified by silica gel column chromatography (chloroform/methanol = 100/1 to 50/3) to give 6-phenyl-1-propyl-5H-pyrrolo[3,2-d]pyrimidin-2,4(1H,3H)-dione (Compound G: 4.56 g, 38%).
¹H-NMR(270 MHz, DMSO-d₆)δ 12.35 (1H, br s), 10.85 (1H, br s), 7.90 (2H, d, J = 7.3 Hz), 7.44-7.39 (2H, m), 7.32 (1H, t, J = 6.9 Hz), 6.73 (1H, s), 3.80 (2H, t, J = 7.3 Hz), 1.71-1.62 (2H, m), 0.91 (3H, t, J = 7.4 Hz).

The title compound (1.11 g, 78%) was obtained in the same manner as in Step 2 of Reference Example 1 by using Compound G (1.28 g, 4.76 mmol) as a starting material.
¹H-NMR(270 MHz, DMSO-d₆)δ 12.53 (1H, br s), 8.02 (2H, d, J = 6.6 Hz), 7.53-7.47 (3H, m), 7.03 (1H, s), 3.96 (2H, t, J = 7.6 Hz), 2.72 (3H, s), 1.73-1.70 (2H, m), 0.94 (3H, t, J = 7.4 Hz).

### Reference Example 3: 4-Chloro-2-phenyl-7-propyl-2H-pyrazolo[3,4-d]pyrimidin-6(7H)-one (Compound H)

6-Chloro-2,4(1H,3H)-pyrimidindione (11.4 g, 78.0 mmol) prepared by the method described in Heterocycles, (1990) 31, p.1641 was dissolved in dimethylsulfoxide (78 mL), and to the mixture were added potassium carbonate (5.25 g, 39.0 mmol) and iodopropane (11.4 mL, 117 mmol), then the mixture was stirred at 60°C for 1 hour. After 4% aqueous sodium hydroxide (80 mL) was added to the reaction mixture at the same temperature, the mixture was air-cooled to room temperature, and washed with toluene (50 mL x 2). The reaction mixture was adjusted to pH 3 by adding hydrochloric acid, and the resulting precipitate was washed with water and dried to give 6-chloro-1-propyl-2,4(1H,3H)-pyrimidindione (Compound I: 6.81 g, 46%).

Compound I (5.65 g, 30.0 mmol) was suspended in ethanol (30 mL), and to the mixture was added phenylhydrazine (5.91 mL, 60.0 mmol), then the mixture was heated under reflux for 2 hours, and the reaction mixture was concentrated. After to the residue was added water, the resulting precipitate was isolated by filtration and washed with water and then dried to give 6-phenylhydrazino-1-propyl-2,4(1H,3H)-pyrimidindione (Compound J: 4.37 g, 56%).

Phosphorous oxychloride (1.68 mL, 18.0 mmol) was added to N,N-dimethylformamide (3 mL) under ice cooling, and the mixture was stirred at room temperature for 10 minutes. To the mixture was added an N,N-dimethylformamide solution (10 mL) of Compound J (3.90 g, 15.0 mmol), and the mixture was heated under reflux for 30 minutes. The reaction mixture was poured into iced water (100 mL), and the resulting precipitate was collected by filtration. The precipitate was washed with water and dried to obtain a brown solid (4.88 g). The brown solid was washed with ethanol to give 2-phenyl-7-propyl-2H-pyrazolo[3,4-d]pyrimidin-4,6(5H,7H)-dione (Compound K: 2.94 g, 73%).
¹H-NMR(270 MHz, DMSO-d₆)δ 11.11 (1H, br s), 9.20 (1H, s), 7.76 (2H, d, J = 8.3 Hz), 7.57-7.50 (2H, m), 7.38 (1H, t, J = 7.4 Hz), 3.91 (2H, t, J = 7.3 Hz), 1.78-1.67 (2H, m), 0.91 (3H, t, J = 7.4 Hz).

To phosphorous oxychloride (20 mL) were added Compound K (1.50 g, 5.56 mmol) and N,N-diisopropylethylamine (2 drops), and the mixture was heated under reflux for 6 hours. The solvent was evaporated from the reaction mixture. The residue was poured into saturated aqueous sodium hydrogen carbonate (50 mL) under ice cooling, and then the mixture was extracted with chloroform (100 mL x 3). An organic layer was washed with saturated brine (100 mL) and dried over anhydrous magnesium sulfate. The solvent was evaporated from the organic layer under reduced pressure to give the title compound (500 mg, 31%).
¹H-NMR(270 MHz, CDCl₃)δ 8.28 (1H, s), 7.78 (2H, d, J = 6.3 Hz), 7.58-7.52 (2H, m), 7.45 (1H, t, J = 7.3 Hz), 4.16 (2H, t, J = 7.4 Hz), 1.96-1.85 (2H, m), 1.02 (3H, t, J = 7.4 Hz).

### Reference Example 4: 4-Methylthio-2-phenyl-7-propyl-2H-pyrazolo[3,4-d]pyrimidin-6(7H)-one (Compound L)

The title compound (850 mg, 11%) was obtained in the same manner as in Step 2 of Reference Example 1 by using Compound K (7.00 g, 27.0 mmol) prepared in Reference Example 3 as a starting material.

### Reference Example 5: 2-Cyclopentyl-4-methylthio-7-propyl-2H-pyrazolo[3,4-d]pyrimidin-6(7H)-one (Compound M)

Cyclopentylidene carbazic acid tert-butyl ester (39.6 g, 0.200 mol) prepared by the method discribed in J. Org. Chem., (1981) 46, p.5413 was dissolved in a mixed solvent of tetrahydrofurane (150 mL) and methanol (200 mL), and to the mixture was added sodium cyanoborohydride (15.7 g, 0.250 mol), and then the mixture was stirred at room temperature for 1 hour. The solvent was evaporated from the reaction mixture under reduced pressure. To the residue was added diethyl ether (500 mL), and 0.5 mol/L hydrochloric acid (450 mL) was slowly dropped to the mixture under ice cooling. The resulting mixture was stirred at room temperature for 1 hour. The diethyl ether layer was separated. To the aqueous layer was added potassium carbonate to adjust the pH at 8, and the mixture was extracted with ethyl acetate (200 mL x 3). The diethyl ether layer and the ethyl acetate layer were combined, and the mixture was washed with saturated aqueous sodium hydrogen carbonate (200 mL) and saturated brine (200 mL), then dried over anhydrous magnesium sulfate, and then concentrated to give tert-butyl 3-cyclopentyl carbazate (Compound N: 37.6 g, 100%).

A mixture of Compound N (20.0 g, 0.100 mol) and (ethoxymethylene)cyanoacetic acid ethyl ester (16.9 g, 0.1 mmol) was added to ethanol (100 mL), and the mixture was heated under reflux overnight. After air-cooling to room temperature, the solvent was evaporated under reduced pressure, and 8 mol/L hydrogen chloride/ethanol (100 mL) was added to the residue, then the mixture was heated under reflux for 1 hour. After air-cooling to room temperature, the mixture was concentrated, and 10% hydrochloric acid was added to the residue until the residue was dissolved. Furthermore, the reaction mixture was adjusted to pH 8 with a 10 mol/L sodium hydroxide solution, and the mixture was extracted with chloroform (100 mL x 3). The organic layer was washed with saturated brine (100 mL) and dried over anhydrous magnesium sulfate, then purified by silica gel column chromatography (hexane/ethyl acetate = 4/1 to 3/1) to give ethyl 3-amino-1-cyclopentyl-1H-pyrazole-4-carboxylate (Compound O: 6.69 g, 30%).

A mixture of Compound O (3.35 g, 15.0 mmol) and 4-methoxybenzyl isocyanate (4.89 g, 30.0 mmol) prepared by the method described in J. Chem. Soc. Perkin Trans 1, (1995) p.2783 were added to toluene (30 mL). After to the mixture was added triethylamine (0.695 mL, 5.00 mmol), the mixture was heated under reflux for 2 overnights. After air-cooling, the solvent was evaporated under reduced pressure. Then, the residue was purified by silica gel column chromatography (chloroform/ethyl acetate = 1/1) to give ethyl 1-cylcopentyl-3-(4-methoxybenzylureido)-1H-pyrazole-4-carboxylate (Compound P: 5.79 g, 100%).

Sodium (460 mg, 20.0 mmol) was dissolved in ethanol (60 mL), and to the solution was added an ethanol solution (20 mL) of Compound P (5.79 g, 15.0 mmol), then the mixture was heated under reflux for 1 hour. After the reaction mixture was air-cooled, the solvent was evaporated under reduced pressure. Then, water was added to the residue until the residue was dissolved. Furthermore, the reaction mixture was adjusted to pH 3 with 4 mol/L hydrochloric acid, and resulting precipitate was isolated by filtration. The precipitate was washed with water and dried to give 2-cyclopentyl-5-(4-methoxybenzyl)-2H-pyrazolo[3,4-d]pyrimidin-4,6(5H,7H)-dione (Compound Q: 5.10 g, 100%).

To a solution of Compound Q (5.10 g, 15.0 mmol) in N,N-dimethylformamide solution (60 mL) was added potassium carbonate (2.07 g, 15.0 mmol), and the mixture was stirred at room temperature for 1 hour. To the reaction mixture was added iodopropane (2.19 mL, 22.0 mmol), and the mixture was further stirred at room temperature for 3.5 hours. After the solvent was evaporated from the reaction mixture under reduced pressure, water (100 mL) was added to the residue. The mixture was neutralized with 4 mol/L hydrochloric acid, and the mixture was extracted with chloroform (100 mL x 3). The organic layer was washed with saturated brine (100 mL) and dried over anhydrous magnesium sulfate, and then purified by silica gel column chromatography (hexane/ethyl acetate = 3/1) to give 2-cyclopentyl-5-(4-methoxybenzyl)-7-propyl-2H-pyrazolo[3,4-d]pyrimidin-4,6(5H,7H)-dione (Compound R: 4.96 g, 86%).

Compound R (4.62 g, 12.0 mmol) was dissolved in a mixed solvent of acetonitrile (50 mL) and water (5 mL), and to the mixture was added di-ammonium cerium (IV) nitrate (13.2 g, 24.0 mmol), then the mixture was heated under reflux for 2 hours. After the reaction mixture was air-cooled, the solvent was evaporated under reduced pressure. To the residue were added chloroform (100 mL) and methanol (5 mL), then inorganic salts was removed by Florisil filtration (chloroform/methanol = 20/1). The reaction mixture was concentrated and purified by silica gel column chromatography (chloroform to chloroform/methanol = 25/1) to give 2-cyclopentyl-7-propyl-2H-pyrazolo[3,4-d]pyrimidin-4,6(5H,7H)-dione (Compound S: 2.67 g, 85%).
¹H-NMR(270 MHz, DMSO-d₆)δ 10.87 (1H, br s), 8.44 (1H, s), 4.71 (1H, m), 3.80 (2H, t, J = 7.0 Hz), 2.10-1.95 (2H, m), 1.95-1.80 (2H, m), 1.75-1.70 (2H, m), 1.70-1.50 (4H, m), 0.86 (3H, t, J = 7.4 Hz).

The title compound (2.18 g, 75%) was obtained in the same manner as in Step 2 of Reference Example 1 by using Compound S (2.62 g, 10.0 mmol) as a starting material.
¹H-NMR(270 MHz, CDCl₃)δ 7.73 (1H, s), 4.63 (1H, m), 4.06 (2H, t, J = 7.4 Hz), 2.66 (3H, s), 2.25-2.15 (2H, m), 2.15-2.00 (2H, m), 2.00-1.70 (6H, m), 0.97 (3H, t, J = 7.4 Hz).

### Reference Example 6: 7-Methylthio-2-phenyl-4-propyl-2H-pyrazolo[4,3-d]pyrimidin-5(4H)-one (Compound T)

Compound E (2.34 g, 10.9 mmol) prepared in Reference Example 2 was suspended in chloroform (50 mL), and a chloroform solution (5 mL) of bromine (0.618 mL, 12 mL) was added to the suspension. Then, the mixture was stirred at 60°C for 30 minutes. After the solvent was evaporated from the reaction mixture under reduced pressure, the residue was reslurried in diethyl ether to give 6-bromomethyl-5-nitro-1-propyl-2,4(1H,3H)-pyrimidindione (Compound U: 2.50 g, 78%).

Compound U (2.34 g, 8.00 mmol) was suspended in ethyl acetate (40 mL), and to the mixture was added aniline (0.729 mL, 16.0 mmol), then the mixture was heated under reflux 2 overnights. After the reaction mixture was air-cooled, the solvent was evaporated under reduced pressure. The residue was purified by silica gel column chromatography (chloroform to chloroform/methanol = 25/1) and followed by reslurry in ethyl acetate to give 2-phenyl-4-propyl-2H-pyrazolo[4,3-d]pyrimidin-5,7(4H,6H)-dione 1-oxide (Compound V: 740 mg, 32%).

Compound V (740 mg, 2.59 mmol) was suspended in ethanol (10 mL), and to the mixture was added 10% palladium-carbon (100 mg), then the mixture was stirred under a hydrogen atmosphere at room temperature for 1 hour. The reaction mixture was filtered, and the precipitate was washed with methanol. The filtrate and the washings were combined and concentrated, and then the residue was purified by silica gel column chromatography (chloroform to chloroform/methanol = 50/1) to give 2-phenyl-4-propyl-2H-pyrazolo[4,3-d]pyrimidin-5,7(4H,6H)-dione (Compound W: 180 mg, 26%).
¹H-NMR(270 MHz, DMSO-d₆)δ 11.28 (1H, br s), 8.81 (1H, s), 7.93 (2H, d, J = 7.6 Hz), 7.61-7.59 (2H, m), 7.43 (1H, t, J =7.3 Hz), 3.77 (2H, t, J = 7.4 Hz), 1.76-1.62 (2H, m), 0.92 (3H, t, J = 7.4 Hz).

The title compound (90.0 mg, 50%) was obtained in the same manner as in Step 2 of Reference Example 1 by using Compound W (162 mg, 0.600 mmol) as a starting material.
¹H-NMR(270 MHz, DMSO-d₆)δ 8.88 (1H, s), 7.95 (2H, d, J = 8.3 Hz), 7.59 (2H, dd, J = 8.3, 7.3 Hz), 7.46 (1H, t, J = 7.3 Hz), 3.85 (2H, t, J = 7.4 Hz), 2.59 (3H, s), 1.75-1.67 (2H, m), 0.92 (3H, t, J = 7.4 Hz).

### Reference Example 7: 2-Cyclopentyl-7-methylthio-4-propyl-2H-pyrazolo[4,3-d]pyrimidin-5(4H)-one (Compound X)

Compound E (8.52 g, 40.0 mmol) prepared in Reference Example 2 was dissolved in N,N-dimethylformamide (160 mL), and to the mixture was added 60% sodium hydride (2.00 g, 50.0 mmol), then the mixture was stirred at room temperature for 1 hour. The reaction mixture was cooled to 0°C, and 4-methoxybenzylchloride (5.97 mmol, 44.0 mmol) was slowly dropped to the reaction mixture. Then, the mixture was further stirred at room temperature for 1 hour. After the solvent was evaporated from the reaction mixture under reduced pressure, water (400 mL) was added to the residue, and the mixture was neutralized with 4 mol/L hydrochloric acid. Then the mixture was extracted with chloroform (200 mL x 2). The organic layer was washed with saturated brine (100 mL) and dried over anhydrous magnesium sulfate, and then purified by silica gel column chromatography (hexane/ethyl acetate = 3/1 to 1/1) to give 3-(4-methoxybenzyl)-5-nitro-6-methyl-1-propyl-2,4(1H,3H)-pyrimidindione (Compound Y: 9.63 g, 72%).

Compound Y (9.58 g, 28.8 mmol) was dissolved in tetrahydrofuran (120 mL), and to the mixture was added 60% sodium hydride (1.44 g, 36.0 mmol), then the mixture was stirred at room temperature for 30 minutes. The reaction mixture was cooled to 0°C, and bromine (1.63 mL, 31.6 mmol) was slowly dropped to the reaction mixture, and then the mixture was further stirred at room temperature for 1.5 hours. After the solvent was evaporated from the reaction mixture under reduced pressure, to the residue was added saturated aqueous sodium hydrogen carbonate (100 mL), and the mixture was neutralized with 4 mol/L hydrochloric acid. Then the mixture was extracted with chloroform (200 mL x 2), and the organic layer was washed with saturated brine (100 mL) and dried over anhydrous magnesium sulfate. The solvent was evaporated under reduced pressure to give 6-bromomethyl-3-(4-methoxybenzyl)-5-nitro-1-propyl-2,4(1H,3H)-pyrimidindione (Compound Z: 7.72 g, 65%).

Compound Z (7.64 g, 18.5 mmol) was dissolved in ethyl acetate (180 mL), and the mixture was added cyclopentylamine (4.03 mL, 40.8 mmol) at 0°C, then the mixture was stirred at room temperature overnight. The reaction mixture was filtered and the filtrate was concentrated, then the residue was purified by silica gel column chromatography (hexane/ethyl acetate = 4/1) to give 6-(cyclopentylamino)methyl-3-(4-methoxybenzyl)-5-nitro-1-propyl-2,4(1H,3H)-pyrimidindione (Compound AA: 3.60 g, 47%).

Compound AA (3.60 g, 8.65 mmol) was dissolved in ethanol (100 mL), and the mixture was heated under reflux overnight. After the reaction mixture was air-cooled to room temperature, the solvent was evaporated under reduced pressure. The residue was purified by silica gel column chromatography (chloroform to chloroform/ethyl acetate = 5/1) to give 2-cyclopentyl-6-(4-methoxybenzyl)-4-propyl-2H-pyrazolo[4,3-d]pyrimidin-5,7(4H,6H)-dione 1-oxide (Compound BB: 2.27 g, 66%).

Compound BB (2.27 g, 5.70 mmol) was dissolved in ethanol, and to the solution was added 10% palladium-carbon (200 mg), then the mixture was stirred under a hydrogen atmosphere at room temperature for 6 hours. After the reaction mixture was filtered, the precipitate was washed with methanol. The filtrate and the washings were combined and concentrated, then the residue was purified by silica gel column chromatography (chloroform) to give 2-cyclopentyl-6-(4-methoxybenzyl)-4-propyl-2H-pyrazolo[4,3-d]pyrimidin-5,7(4H,6H)-dione (Compound CC: 2.12 g, 98%).

Compound CC (2.12 g, 5.50 mmol) was dissolved in a mixed solvent of acetonitrile (27 mL) and water (3 mL), and to the mixture was added di-ammonium cerium (IV) nitrate (3.29 g, 6.00 mmol), then the mixture was heated under reflux for 2.5 hours. Further, to the reaction mixture was added Di-ammonium cerium (IV) nitrate (3.29 g, 6.00 mmol), and the mixture was heated under reflux for 30 minutes. After the reaction mixture was air-cooled, and the solvent was evaporated under reduced pressure. Then, to the residue were added chloroform (100 mL) and methanol (5 mL). The mixture was filtered through Celite, and the precipitate was washed with chloroform/methanol (20/1). The filtrate and the washings were combined and concentrated, and the residue was purified by silica gel column chromatography (chloroform to chloroform/methanol = 50/1) to give 2-cyclopentyl-4-propyl-2H-pyrazolo[4,3-d]pyrimidin-5,7(4H,6H)-dione (Compound DD: 820 mg, 57%).
¹H-NMR(270 MHz, DMSO-d₆)δ 11.04 (1H, br s), 8.09 (1H, s), 4.79 (1H, m), 3.68 (2H, t, J = 7.2 Hz), 2.16-2.07 (2H, m), 1.99-1.89 (2H, m), 1.80-1.77 (2H, m), 1.71-1.57 (4H, m), 0.87 (3H, t, J = 7.4 Hz).

The title compound (508 mg, 56%) was obtained in the same manner as in Step 2 of Reference Example 1 by using Compound DD (820 mg, 3.13 mmol) as a starting material.
¹H-NMR(270 MHz, CDCl₃)δ 7.28 (1H, s), 4.80 (1H, m), 3.87 (2H, t, J = 7.6 Hz), 2.67 (3H, s), 2.35-2.15 (2H, m), 2.15-2.00 (2H, m), 2.00-1.85 (2H, m), 1.85-1.65 (4H, m), 0.98 (3H, t, J = 7.4 Hz).

### Test Example 1: Insulin secretion stimulating activity in cultured β cells

Pancreatic β cell line MIN6 cells reported by Miyazaki, et al. [see Endocrinology, (1990) 127, p.126-131] exhibit characteristics of insulin content and insulin secretion amount by stimulation with glucose similar to those of pancreatic β cells in vivo, and well preserves characteristics of pancreatic β cells in vivo from a view point that it shows increase of insulin secretion in a glucose concentration-dependent manner [the above reference and Diabetologia, (1993) 36, p.1139-1145]. In MIN6 cells, insulin secretion increase responding to a sulfonylurea agents, such as Glybenclamide, which are used as a therapeutic agent for diabetes (Cellular Signaling, (1993) 5, p.777-786). Therefore, the above-mentioned culture of MIN6 cells and an insulin secretion test using the MIN6 cells were performed according to the method described in Diabetologia, (1993) 36, p.1139-1145.

The effects of a compound on an insulin secretion activity under the presence of 14.5 mmol/L glucose was determined by measuring insulin amount in cell-culture supernatant collected as follows:

MIN6 cells cultured in a 24-well plate were washed twice with 1 mL of buffer solution A (pH 7.3.) containing 2 mmol/L glucose. Buffer solution A consists of 119 mmol/L sodium chloride, 4.74 mmol/L potassium chloride, 2.54 mmol/L calcium chloride, 1.19 mmol/L magnesium sulfate, 1.19 mmol/L potassium dihydrogen phosphate, 10 mmol/L 2-[4-(2-hyroxyethyl)-1-piperazinyl]ethane sulfonic acid and 0.1% bovine serum albumin. Then, the cells were incubated in 1 mL of buffer solution A containing 2 mmol/L glucose at 37°C for 45 minutes. After the incubation, the culture supernatant was exchanged with 0.9 mL of buffer solution A containing each test compound at various concentrations and 2 mmol/L glucose. The cells were further incubated at 37°C for 15 minutes. Then, the MIN6 cells were stimulated with glucose by adding 0.1 mL of buffer solution A containing 127 mmol/L glucose (a final glucose concentration: 14.5 mmol/L). After the stimulation, the cells were further incubated at 37°C for 45 minutes, and the supernatant was collected.

Antibody reactive insulin secreted in the culture supernatant was diluted with a phosphate buffer solution containing 1% bovine serum albumin, 0.1% Tween 20, 0.12% ethylenediaminetetraacetic acid (EDTA) disodium salt and 0.1% sodium azide, and then quantitatively measured by enzyme immunoassay or radioimmunoassay. The insulin levels were indicated as the amount of human insulin (ng/mL). The results are indicated by averages (avg) and standard errors (se) of 3 to 4 tests.

The results are shown in Table 2.

**Table 2**

| Compound No. | Drug concentration (µmol/L) | Insulin secretion content (ng/mL) | |
|---|---|---|---|
| | | ave | se |
| None | - | 148.4 | 4.8 |
| 1 | 1.0 | 204.3 | 6.1 |
| 2 | 1.0 | 187.1 | 2.6 |
| 3 | 1.0 | 213.2 | 9.1 |
| 4 | 1.0 | 212.1 | 1.9 |
| 5 | 1.0 | 190.9 | 3.0 |
| 6 | 1.0 | 172.5 | 3.7 |
| 7 | 1.0 | 224.8 | 11.6 |
| 8 | 1.0 | 183.9 | 8.3 |
| 9 | 1.0 | 174.3 | 0.7 |
| 10 | 1.0 | 184.7 | 1.6 |

As shown in Table 2, it is revealed that the fused pyrimidine derivatives according to the present invention have remarkable activities for stimulating insulin secretion.

### Industrial Applicability

According to the present invention, fused pyrimidine derivatives having an insulin secretion stimulating activity and pharmaceutically acceptable salts thereof are provided.

## Claims

1. A fused pyrimidine derivative or a pharmaceutically acceptable salt thereof represented by Formula (I): 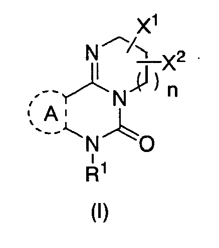 {wherein R¹ represents a hydrogen atom, substituted or unsubstituted lower alkyl, substituted or unsubstituted aralkyl, substituted or unsubstituted aryl, or substituted or unsubstituted aromatic heterocyclic group; n represents an integer of 0 to 3; X¹ and X² may be the same or different and each represents a hydrogen atom, substituted or unsubstituted lower alkyl, substituted or unsubstituted aralkyl, substituted or unsubstituted aryl, or substituted or unsubstituted aromatic heterocyclic group; and formula (II): 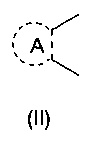 represents formula (III): 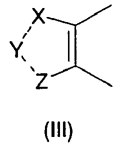 [wherein X--Y--Z represents R²C=CR³-NR⁴ (wherein R², R³ and R⁴ may be the same or different and each represents a hydrogen atom, substituted or unsubstituted lower alkyl, substituted or unsubstituted aralkyl, substituted or unsubstituted aryl, or substituted or unsubstituted aromatic heterocyclic group), R²C=N-NR⁴ (wherein R² and R⁴ have the same meanings as defined above, respectively), R⁴N-CR³=CR² (wherein R², R³ and R⁴ have the same meanings as defined above, respectively), or R⁴N-N=CR² (wherein R² and R⁴ have the same meanings as defined above, respectively)] or formula (IV): 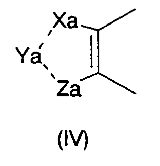 [wherein Xa--Ya--Za represents R²HC-NR³-CHR⁴ (wherein R², R³ and R⁴ have the same meanings as defined above, respectively), R²HC-NR³-NH (wherein R² and R³ have the same meanings as defined above, respectively), or NH-NR³-CHR⁴ (wherein R³ and R⁴ have the same meanings as defined above, respectively)]).

2. The fused pyrimidine derivative or a pharmaceutically acceptable salt thereof according to claim 1, wherein R¹ is substituted or unsubstituted lower alkyl.

3. The fused pyrimidine derivative or a pharmaceutically acceptable salt thereof according to claim 1 or 2, wherein R³ is substituted or unsubstituted aryl, or substituted or unsubstituted lower alkyl.

4. The fused pyrimidine derivative or a pharmaceutically acceptable salt thereof according to any one of claims 1 to 3, wherein X¹ is substituted or unsubstituted aralkyl and X² is a hydrogen atom.

5. A Pharmaceutical composition comprising the fused pyrimidine derivative or a pharmaceutically acceptable salt thereof according to any one of claims 1 to 4 as an active ingredient.

6. A therapeutic agent for diabetes comprising the fused pyrimidine derivative or a pharmaceutically acceptable salt thereof according to any one of claims 1 to 4 as an active ingredient.

7. A preventive and/or therapeutic agent for diabetic complications comprising the fused pyrimidine derivative or a pharmaceutically acceptable salt thereof according to any one of claims 1 to 4 as an active ingredient.

8. A blood glucose-lowering agent comprising the fused pyrimidine derivative or a pharmaceutically acceptable salt thereof according to any one of claims 1 to 4 as an active ingredient.

9. An insulin secretagogue comprising the fused pyrimidine derivative or a pharmaceutically acceptable salt thereof according to any one of claims 1 to 4 as an active ingredient.

10. Use of the fused pyrimidine derivative or a pharmaceutically acceptable salt thereof according to any one of claims 1 to 4 for a manufacture of a therapeutic agent for diabetes.

11. Use of the fused pyrimidine derivative or a pharmaceutically acceptable salt thereof according to any one of claims 1 to 4 for a manufacture of a preventive and/or therapeutic agent for diabetic complications.

12. Use of the fused pyrimidine derivative or a pharmaceutically acceptable salt thereof according to any one of claims 1 to 4 for a manufacture of a blood glucose-lowering agent.

13. Use of the fused pyrimidine derivative or a pharmaceutically acceptable salt thereof according to any one of claims 1 to 4 for a manufacture of an insulin secretagogue.

14. A method for treating diabetes, which comprises administering an effective amount of the fused pyrimidine derivative or a pharmaceutically acceptable salt thereof according to any one of claims 1 to 4.

15. A method for preventing and/or treating diabetic complications, which comprises administering an effective amount of the fused pyrimidine derivative or a pharmaceutically acceptable salt thereof according to any one of claims 1 to 4.

16. A method for lowering blood glucose levels, which comprises administering an effective amount of the fused pyrimidine derivative or a pharmaceutically acceptable salt thereof according to any one of claims 1 to 4.

17. A method for stimulating secretion of insulin, which comprises administering an effective amount of the fused pyrimidine derivative or a pharmaceutically acceptable salt thereof according to any one of claims 1 to 4.
